# EUROPEAN PATENT APPLICATION

(11) **EP 4 230 179 A1**
(43) Date of publication of application: **23.08.2023**
(21) Application number: 23157849.3
(22) Date of filing: 21.02.2023
(51) Int. Cl.: A61F 2/30, A61B 34/10

(54) **USING NUMERICAL METHODS TO OPTIMIZE IMPLANT DESIGN**

(30) Priority: 21.02.2022 US 202263312212 P
(71) Applicant: Zimmer, Inc., Warsaw, IN 46580 (US)
(72) Inventor: FAVRE, Philippe, 8047 Zurich (CH); SON, Yang Wook, Granger, 46530 (US); MUERI, Christine Mirja, 8405 Winterthur (CH)
(74) Representative: Mays, Julie

(57) **Abstract**

Disclosed herein are systems and methods for manufacturing a prosthetic. The systems and methods can include receiving patient data and determining optimal design parameters (408) for the prosthetic using the patient specific data and a machine learning model (702). The optimal design parameters can be exported so that the prosthetic can be manufactured.

## Description

### CLAIM OF PRIORITY

This application claims the benefit of U.S. Provisional Patent Application Serial No. 63/312,212, filed on February 21, 2022, the benefit of priority of which is claimed hereby, and which is incorporated by reference herein in its entirety.

### FIELD OF THE DISCLOSURE

The present disclosure relates generally to surgical implants and methods of design thereof. More specifically, the present disclosure relates to using artificial intelligence (AI) in conjunction with finite element analysis (FEA) to design patient specific implants and methods of use thereof.

### BACKGROUND

Traditional implants used in joint replacement possess a stem to ensure fixation of the component with the cortex of a bone canal. Implantation of the implants generally requires that the articulation be resected and then the bone canal reamed or rasped to prepare the bone to receive a stem of the implant. To implant a component of the implant, the component is driven into the bone canal using a hammer or other impact tool. Much of the preparation of the bone and insertion of a stem into the bone canal can be eliminated and/or reduced, thereby resulting in less bone removal, using stemless implants as disclosed herein.

### SUMMARY

The following, non-limiting examples, detail certain aspects of the present subject matter to solve the challenges and provide the benefits discussed herein, among others.

Example 1 is a method for manufacturing a prosthetic, the method comprising: receiving, at a computing device, patient specific data; determining, by the computing device, optimal design parameters for the prosthetic using the patient specific data and a model; and exporting, by the computing device, the optimal design parameters.

In Example 2, the subject matter of Example 1 optionally includes where determining the optimal design parameters includes determining a size of a placement fixture.

In Example 3, the subject matter of any one or more of Examples 1-2 optionally include where determining the optimal design parameters includes determining a number of placement fixtures.

In Example 4, the subject matter of any one or more of Examples 1-3 optionally include where determining the optimal design parameters includes determining a shape of a placement fixture.

In Example 5, the subject matter of any one or more of Examples 1-4 optionally include where determining the optimal design parameters includes determining a location of a placement fixture.

In Example 6, the subject matter of any one or more of Examples 1-5 optionally include where determining the optimal design parameters includes iterating over a plurality of guesses for the optimal design parameters.

In Example 7, the subject matter of any one or more of Examples 1-6 optionally include generating a plurality of designs for the prosthetic, each of the plurality of designs including a different permutation of design parameters.

In Example 8, the subject matter of any one or more of Examples 1-7 optionally include wherein receiving the patient specific data comprises receiving a scan of a patient's anatomy; and the method further comprises extracting design constraints for the prosthetic from the scan.

In Example 9, the subject matter of any one or more of Examples 1-8 optionally include wherein exporting the optimal design parameters includes transmitting the optimal design parameters to an automated manufacturing device.

Example 10 is a system for manufacturing a prosthetic, the system comprising: at least one processor; and at least one memory storing instructions that, when executed by the at least one processor, cause the at least one processor to perform actions comprising: receiving patient specific data, determining optimal design parameters for the prosthetic using the patient specific data and a machine learning model, and exporting the optimal design parameters.

In Example 11, the subject matter of Example 10 optionally includes where determining the optimal design parameters includes determining a size of a placement fixture.

In Example 12, the subject matter of any one or more of Examples 10-11 optionally include where determining the optimal design parameters includes determining a number of placement fixtures.

In Example 13, the subject matter of any one or more of Examples 10-12 optionally include where determining the optimal design parameters includes determining a size and shape of a placement fixture.

In Example 14, the subject matter of any one or more of Examples 10-13 optionally include wherein the actions further comprise generating a plurality of designs for the prosthetic, each of the plurality of designs including a different permutation of design parameters.

In Example 15, the subject matter of any one or more of Examples 10-14 optionally include wherein receiving the patient specific data comprises receiving a scan of a patient's anatomy; and the actions further comprise extracting design constraints for the prosthetic from the scan.

Example 16 is a method for generating a machine learning model used to design a prosthetic, the method comprising: receiving, by a computing device, a plurality of input parameters, each of the plurality of input parameters corresponding to a parameter of at least one of a plurality of prosthetic designs; generating, by the computing device, an initial guess for the machine learning model based on the plurality of input parameters; optimizing, by the computing device, parameters of the machine learning model using the initial guess for the machine learning model and the plurality of input parameters; and exporting, by the computing device, the machine learning model having optimized parameters.

In Example 17, the subject matter of Example 16 optionally includes wherein optimizing the parameters of the machine learning model comprises training the machine learning model using a set of finite element analyses performed on models of the prosthetic and at least a subset of the input parameters.

In Example 18, the subject matter of any one or more of Examples 16-17 optionally include wherein the plurality of input parameters comprises patient data for a plurality of patients.

In Example 19, the subject matter of any one or more of Examples 16-18 optionally include dividing the plurality of input data into a training subset used in optimizing the parameters of the machine learning model and a validation subset used for confirming a validity of the machine learning model.

In Example 20, the subject matter of any one or more of Examples 16-19 optionally include wherein receiving the plurality of input parameters comprises: receiving a scan of a patient's anatomy for a plurality of patients; and extracting at least one of the input parameters from the scan of the patient's anatomy for each of the plurality of patients.

Example 21 is at least one computer-readable medium comprising instructions to perform any of the methods of Examples 1-9 and 16-20.

Example 22 is an apparatus comprising means for performing any of the methods of Examples 1-9 and 16-20.

In Example 23, the systems and methods of any one or any combination of Examples 1 - 22 can optionally be configured such that all elements or options recited are available to use or select from.

### BRIEF DESCRIPTION OF THE FIGURES

In the drawings, which are not necessarily drawn to scale, like numerals can describe similar components in different views. Like numerals having different letter suffixes can represent different instances of similar components. The drawings illustrate generally, by way of example, but not by way of limitation, various embodiments discussed in the present document.
FIGS. 1A and 1B show a stemless implant in accordance with at least one example of this disclosure.
FIG. 2 shows a fixation component in accordance with at least one example of this disclosure.
FIG. 3 shows a fixation component in accordance with at least one example of this disclosure.
FIG. 4 shows a method in accordance with at least one example of this disclosure.
FIG. 5 shows a table of inputs in accordance with at least one example of this disclosure.
FIG. 6 shows a table of outputs in accordance with at least one example of this disclosure.
FIG. 7 shows a method in accordance with at least one example of this disclosure.
FIG. 8 shows a schematic of a computing device in accordance with at least one example of this disclosure.

Corresponding reference characters indicate corresponding parts throughout the several views. The exemplifications set out herein illustrate exemplary embodiments of the disclosure, and such exemplifications are not to be construed as limiting the scope of the disclosure in any manner.

### DETAILED DESCRIPTION

Various metaphyseal implants do not have a stem. Fixation of these stemless implants can be provided by design features that anchor the implant to bone, such as trabecular bone. Thus, the implants can be implanted without contact with the cortex or the requirements that the bone canal be reamed or rasped.

Fixation of stemless implants can be important to the clinical success of such devices. The stemless implant can act as an anchor in the bone. Stated another way, the stemless implant can keep the implant in position and minimizes motion of a head, tray, or bearing relative to the host bone in vivo.

As disclosed herein, stemless implants can come in a multitude of different designs and with features that provide fixation stability. For example, fixation features can include various numbers of fins, with the fins being similar in size and shape or the various fins having different shapes, lengths, widths, and depths of penetration into trabecular bone. In some instances, the fixation can be provided by a helical structure and central post or screws. Stemless implants can then be connected to a second component, such as a head, tray, or bearing, that reconstructs the damaged part of an articulation (e.g., the humeral or femoral head).

Fixation features can be designed to fit within the bones found in the general population to provide sufficient fixation, while at the same time preserving as much bone as possible. As disclosed herein, optimization of the fixation features and fins (e.g., the number of fins, shape, length, width, depth, etc.) can be accomplished using computer simulation in order to maximize implant performance. Performance can be measured as, but is not limited to, primary stability (e.g., as measured by relative motion at the implant/bone interface), stress shielding (e.g., measured by bone strain/stress), and/or bone preservation (e.g., as measured by the volume of bone removed by the implant).

As disclosed herein, fixation features can be designed based on basic mechanical principles, empirical knowledge, and/or surgeon preferences. The geometry of the implant can be designed to fit within the space given by the bone. The implant can be further designed to provide sufficient stability, minimize stress shielding, preserve as much bone as possible, and allow a simple surgical procedure.

As disclosed herein, computational methods can be leveraged to generate designs as well as test those designs via finite element analysis (FEA) to evaluate performance. An optimized design has the potential to provide greatly improved fixation, stress shielding, bone preservation, and therefore the clinical success of such implants can be enhanced. As disclosed herein, the design and testing can include use of simulation methods to design implants that provide increased fixation to the host bone, while fitting the patient.

Design of experiments (DOE), AI, virtual topology, and combinations thereof can be used to generate a large number of different design variations for a given patient. The different design variations may be generated by altering implant dimensions within given boundaries (e.g., physical size of a patient's joint or other anatomical structures). The various designs can then be subjected to FEA and the design showing optimal performance can be selected based on simulation results.

As disclosed herein, the design and manufacturing of a prosthetic can include, receiving patient specific data and determining optimal design parameters for the prosthetic using the patient specific data and numerical methods, such as a machine learning model, DOE or virtual topology. The optimal design parameters can be exported and used to manufacture the prosthetic. For example, the optimal design parameter can be transmitted to an automated manufacturing device.

Determining the optimal design parameters can include determining a size of a placement fixture, determining a number of placement fixture of the prosthetic, and determining a shape of a placement fixture. Determining optimal design parameters can include iterating over a plurality of guesses for the optimal design parameters as well as generating a plurality of designs for the prosthetic, each of the plurality of designs including a different permutation, or iteration, of design parameters.

Generating a machine learning model used to design a prosthetic can include receiving a plurality of input parameters and training a model using data sets from actual patients and/or data sets generated using FEA, by means of, for example, DOE or virtual topology. Each of the plurality of input parameters can correspond to a parameter of at least one of a plurality of prosthetic designs. An initial guess can be generated for the machine learning model based on the plurality of input parameters and parameters of the machine learning model can be optimized using the initial guess for the machine learning model and the plurality of input parameters. The machine learning model having optimized parameters can be exported for used in designing and manufacturing prosthetics.

The above discussion is intended to provide an overview of subject matter of the present patent application. It is not intended to provide an exclusive or exhaustive explanation of the invention. The description below is included to provide further information about the present patent application.

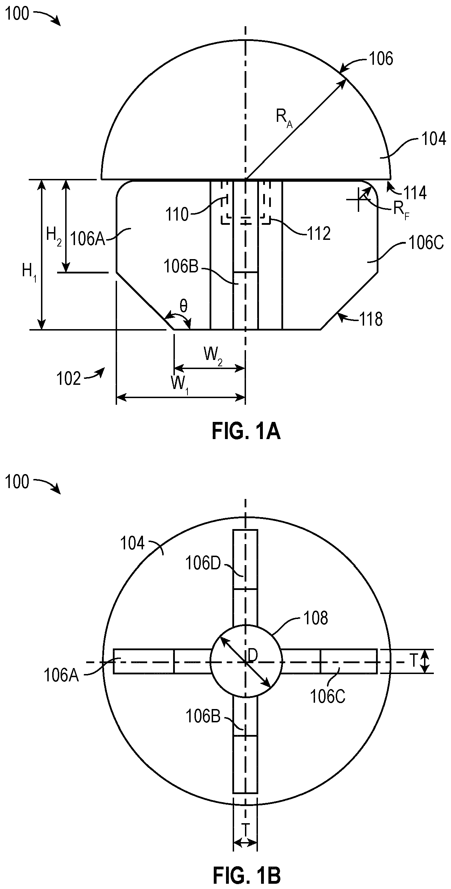

Turning now to the figures, FIGS. 1A and 1B show a stemless implant 100 in accordance with at least one example of this disclosure. Stemless implant 100 can include a fixation component 102 and an articulation component 104. Fixation component 102 can include fins 106 (labeled individually as fins 106A, 106B, 106C, and 106D) that extend from a central portion 108. While FIGS. 1A and 1B shows four fins, fixation component 102 can have any number of fins and the number of fins can be a design parameter as disclosed herein.

Central portion 108 can define a recess 110. Articulation component 104 can include a peg 112 that extends from a surface 114 and have an articulation surface 116 having a radius, *R_{A}*. While FIG. 1A shows articulation surface 116 having a constant radius, the radius can vary to give articulation surface 116 a contoured and/or oval shaped surface. Recess 110 and peg 112 can be sized to mate together thereby securing articulation component 104 to fixation component 102. Still consistent with embodiments disclosed herein, fixation component 102 can include a peg and articulation component 104 can define a corresponding recess to receive the peg.

As disclosed herein, fins 106 can have various dimensions. For example, each of fins 106 can have an overall width, *W₁,* and an overall height, *H₁.* Each of fins 106 can have a secondary width, *W₂*, and a secondary height *H₂*. The various heights and widths can define a fin angle, *θ*. Second width, *H₂* and secondary height *H₂,* can each be zero such that an angled surface 118 extends from central portion 108 to surface 114. Second width, *H₂* and secondary height *H₂,* can be equal and *W₁* and *W₂* can also be equal, thus eliminating angled surface 118. Fins 106 can also have corners that are radiused, such as having a radius *R_{F}*, and/or chambered. Fins 106 can also have a thickness, *T.* Central portion 108 can have a diameter, *D*. Diameter, *D*, can be constant such that central portion 108 is cylindrical and/or vary such that central portion 108, or a portion of central portion 108 is conical.

The various dimensions for fixation component 102 can vary among various designs for prosthetics. The various dimensions for fixation component 102 can vary from one patient to another. Still consistent with embodiments disclosed herein, the various dimensions can differ from one another for the same fixation component. For example, the thickness, *T*, of fins 106A and 106B may be the same and the thickness, *T*, of fins 106C and 106D may differ from one another and that of fins 106A and 106B. Heights, *H₁* and *H₂,* widths, *W₁* and *W₂*, can also very from fin to fin. Fins 106 can be coated with porous material to allow for bone in growth.

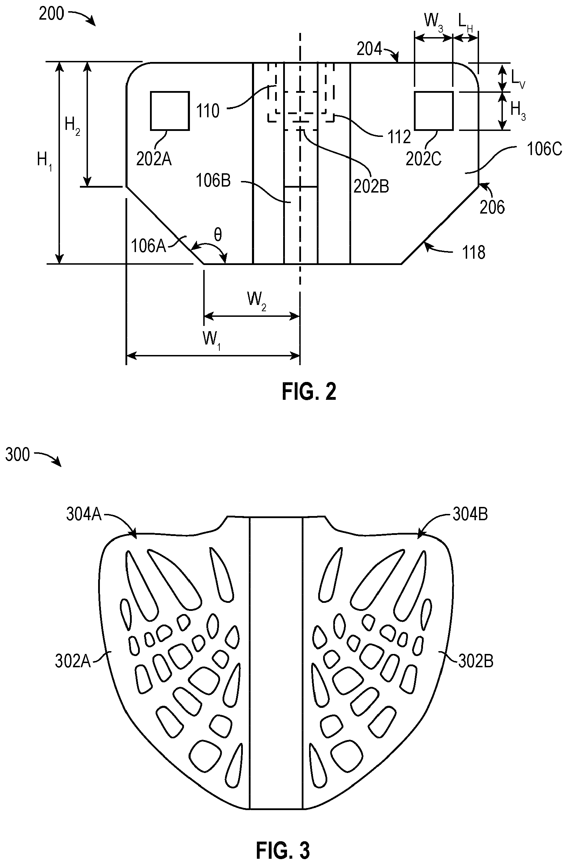

FIG. 2 shows a fixation component 200 in accordance with at least one example of this disclosure. Fixation component 200 can be similar to fixation component 100. As shown in FIG. 2, each of fins 106 can define one more openings 202 (labeled individually as openings 202A, 202B, and 202C). Openings 202 can be rectangular. For example, each of openings 202 can have a width, *W₃,* and a height, *H₃,* that can vary from fin to fin. Each of openings 202 can be located a vertical length, *L_{V},* from a first surface 204 and a horizontal length, *L_{H},* from a second surface 206. While FIG. 2 shows a single opening in each of fins 106, fins 106 can have any number of opens and the openings can be other shapes besides rectangular. For example, any one of or combination of openings 202 circular, oval, triangular, etc.

FIG. 3 shows a fixation component 300 in accordance with at least one example of this disclosure. FIG. 3 can represent a design that can be generated using virtual topology simulations. As shown in FIG. 3, each of fins 302 (labeled individually as fins 302A and 302B) of fixation component 300 can include a plurality of openings 304 (labeled as subset 304A and subset 304B). While FIG. 3 shows subset 304A and subset 304B being the same, subsets 304A and 304B can have a different number of openings and each of the different openings can have a different size and/or shape.

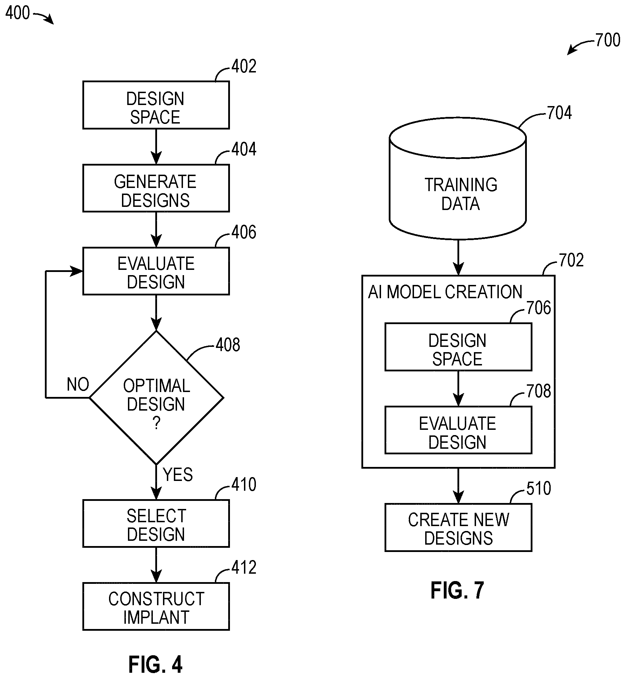

FIG. 4 shows a method 400 for manufacturing a prosthetic in accordance with at least one example of this disclosure. Method 400 can begin at stage 402 where a design space can be defined. Determining the design space can include defining ranges for various parameters for a prosthetic. The various example design parameters (e.g., depth, diameter, fin width, angle, etc.) can be defined as factors to vary, in order to modify the geometry of each fin. The fins can also have holes of various geometric dimensions and position within the fin that can be defined as factors to vary for the analysis as disclosed herein.

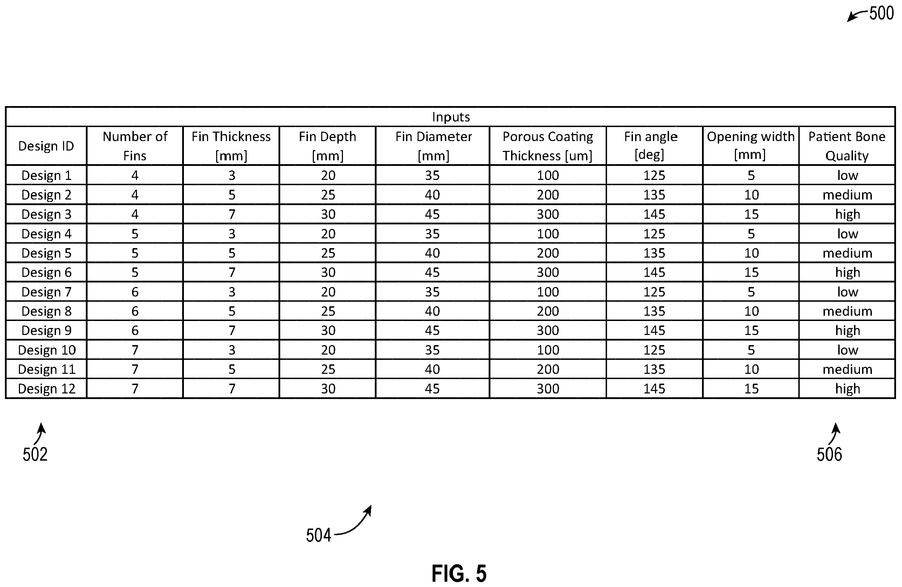

The number of fins can be defined as factors to vary. As disclosed herein, various statistical methods may be used to assign values to each chosen geometric factors within a predefined range to define the design space. By select various ranges for the design parameters, a large number of different designs can be generated (404). FIG. 5 shows a table 500 having example inputs that can be used to define the design space. For example, and as shown in FIG. 5, each design can be assigned a design ID 502. Each design can have various properties that can be inputs 504 to a model as disclosed herein. Inputs 504 can include parameters for a fixation component as well as parameters related to a patient. For example, inputs 504 can include parameters related to bone quality as indicated by reference numeral 506.

As disclosed herein, inputs 504 can be numerical values, such as dimensions related to openings in bone, fin dimensions, etc. Inputs 504 can also be string values. For example, the bone quality can be represented as strings as shown in FIG. 5. The bone quality can also be represented by numeric values as well. For instance, low bone quality could be represented as 1, medium bone quality as 2, etc.

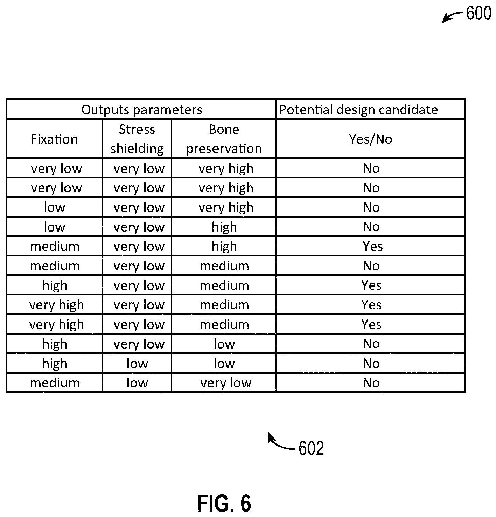

Once the various designs are generated, each of the designs can be tested numerically using FEA (406). The FEA can provide output. FIG. 6 shows a table 600 with example outputs 602. Outputs 602 can be presented as strings or numerical values. For example, as shown in FIG. 6, outputs 602 can be presented as a table (or array) of strings for a surgeon to review.

Outputs 602 can be derived and/or represented by numerical values. For example, very high fixation can be represented as 1, high fixation can be represented as a 2, etc. The fixation can be based on movement of a fixation component as determined during the FEA. Stresses can also be determined during the FEA process. These values may be numerical and converted to strings for presentation to a surgeon as shown in FIG. 6.

As the designs are evaluated determinations can be made as to if the designs are optimal (408). This determination can be made by a computing system as disclosed herein and/or by a surgeon. For example, designs that show low fixation and low bone preservation can be eliminated by the computing system and not presented to the surgeon. While table 500 shows only 12 design options, the data set can include thousands, if not tens or even hundreds of thousands of design options. Thus, the evaluation of designs can include evaluating multiple designs and selecting a preset number of potential optimal designs (410) for presentation to the surgeon.

As part of the design selection and evaluation, implant fit can be provided by keeping the implant boundaries within the available trabecular bone, without contacting the cortical bone. This can establish boundary conditions for the FEA. Therefore, the design space can be defined by the geometry of the internal cortical bone. This can be obtained on a patient specific basis (with a CT scan of the patient for example), or on a more general basis, by using morphological analyses of the population (with ZiBRA for example). This may also be obtained based on demographic data (age, gender, height).

Once a design is selected, the design parameters can be exported for construction of the implant (412). For example, the design parameters for the implant, whether a custom implant or a stock implant, can be exported to a three-dimensional printer, computer numerical controlled machining device, etc. to allow the implant to be constructed.

FIG. 7 shows a method 700 for designing various prosthetic designs in accordance with at least one example of this disclosure. Method 700 can begin with the creation of an AI model (702). Creating the AI model can include inputs from a training data set 704. AI model creation can also include defining a design space 706 and evaluating designs 708, which can be similar to generating a design space and evaluating designs as disclosed with respect to FIG. 4.

The design space can be affected by the surgical procedure. Thus, the AI model input can include the surgical procedure. For example, depending on how the bone is cut, more or less of the intact bone can be included in the design space. Also, implant placement in the bone can dictate how much space is available in each direction. In one embodiment, method 700 can be implemented to design generic implant design (510), based on average bone density and load data. This generic design can be scaled to create different sizes that can fit the population.

As part of the AI model creation, a formula can be generated. The formula can allow the various inputs to be entered. The formula may output a number that corresponds to one of the various designs that have been created. For example, the various inputs can be entered into the model and the model return a number, X. The number X can correspond to a model number of a stock implant. To select the implant one or more lookup tables may be used to select the implant from a database.

The various inputs can be part of a query to a database. Using the inputs, the model can return the implant that had the closest inputs to the various inputs when a stock implant was designed. For example, each of the stock implants will have preset specifications that were used as part of their creation. The specification can be stored in the database as query variables to select a stock implant. The database may be a K-nearest-neighbor (KNN) database. As such, the Euclidean distance between the inputs for a specific patient and specifications for each of the stock designs can be calculated. The specific design with the minimum distance can be returned as the optimal design for a patient.

Part of generating the AI model can include generating an initial guess for parameters of a machine learning model based on the various inputs. The parameters of the machine learning model can be optimized from the initial guess for the machine learning model and the plurality of input parameters. For example, for a given bone size, bone quality, etc. initial guesses for number of fins, fin thickness, fin height, number of holes in fins, etc. can be made and designs created. Those designs, which can be solid models, can be subjected to FEA to confirm fixation, bone preservation, stress shielding, etc.

Training data 704 can be divided into subsets of data for training and testing. A first subset (e.g., a training subset) of training data 704 can be used to build various models of prosthetics that have been tested using FEA. A second subset (e.g., a validation subset) of training data 704 can be used to validate the various models of prosthetics and/or any machine learning models that are generated as disclosed herein.

Training data can include actual patient data that includes fixation data, bone preservation, etc. obtained via x-rays, CT scans, etc. For example, a scan of a patient's anatomy can be received for a plurality of patients. The various input parameters can be extracted from the scan of the patient's anatomy for each of the plurality of patients and saved as part of training data 704. The machine learning model can be exported for later use as needed.

The systems and methods disclosed herein, can be implemented to design a patient specific implant, where the models can identify the best design for a given patient. The design space can be tailored to best fit the trabecular bone volume, or any bone volume, of the patient, based on scan information, such as CT scan information. Further, the CT scan data can be used to gain information on the patient bone quality, and design the implant to profit from the denser bone regions. Also, the load may be tailored to the patient, based on general patient information. For example, loading can be tailored based on does the patient plays sports, does he or she need to carry heavy weights at her/his job, etc. Such implant designs may be manufactured using standard production methods. For designs that may not be producible readily with standard manufacturing methods, 3D printing may be used.

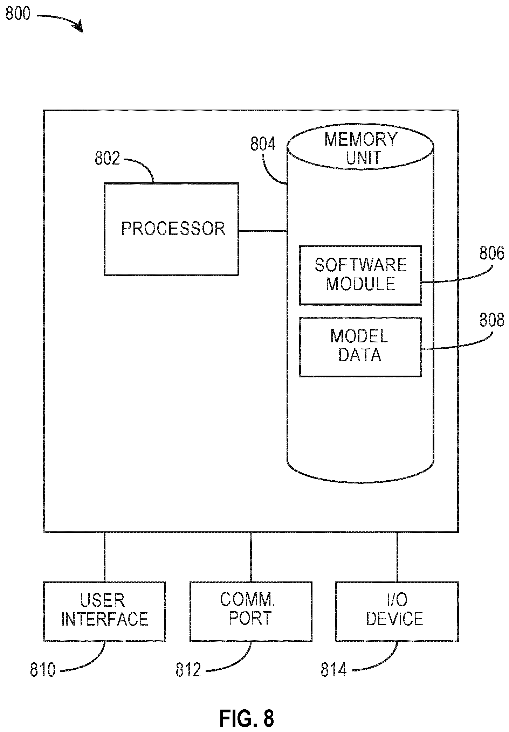

FIG. 8 shows an example schematic of computing device 800 in accordance with at least one example of this disclosure. As shown in FIG. 8, computing device 800 can include a processor 802 and a memory 804. Memory unit 804 can include a software module 806 and model data 808. While executing on processor 802, software module 806 can perform processes for generating AI models, generating prosthetic designs, performing FEA on designs, etc., including, for example, one or more stages included in methods 400 and 700 described below with respect to FIGS. 4 and 7. As disclosed herein, model data 808 can include training data, such as training data 704, design parameters used as inputs, databases, such as KNN databases, models generated using the various designs, etc. as disclosed herein. Computing device 800 can also include a user interface 810, a communications port 812, and an input/output (I/O) device 814.

User interface 810 can include any number of devices that allow a user to interface with computing device 800. Non-limiting examples of user interface 810 can include a keypad, a microphone, a display (touchscreen or otherwise), etc.

Communications port 812 may allow computing device 800 to communicate with various information sources and devices, such as, but not limited to, remote computing devices such as servers or other remote computers. For example, remote computing devices may maintain data, such as model data, that can be retrieved by computing device 800 using communications port 812. Non-limiting examples of communications port 812 can include, Ethernet cards (wireless or wired), Bluetooth^{®} transmitters and receivers, near-field communications modules, etc.

I/O device 814 can allow computing device 800 to receive and output information. Non-limiting examples of I/O device 814 can include, a camera (still or video), fingerprint or other biometric scanners, etc. For example, I/O device 814 may allow computing device 800 to directly receive patient data from a CT scanning device, x-ray machine, etc.

### NOTES

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other embodiments can be used, such as by one of ordinary skill in the art upon reviewing the above description. The Abstract is provided to comply with 37 C.F.R. § 1.72(b), to allow the reader to quickly ascertain the nature of the technical disclosure. It is submitted with the understanding that it will not be used to interpret or limit the scope or meaning of the claims. Also, in the above Detailed Description, various features can be grouped together to streamline the disclosure. This should not be interpreted as intending that an unclaimed disclosed feature is essential to any claim. Rather, inventive subject matter can lie in less than all features of a particular disclosed embodiment. Thus, the following claims are hereby incorporated into the Detailed Description as examples or embodiments, with each claim standing on its own as a separate embodiment, and it is contemplated that such embodiments can be combined with each other in various combinations or permutations. The scope of the invention should be determined with reference to the appended claims, along with the full scope of equivalents to which such claims are entitled.

## Claims

1. A method for manufacturing a prosthetic, the method comprising:
receiving, at a computing device, patient specific data;
determining, by the computing device, optimal design parameters for the prosthetic using the patient specific data and a machine learning model; and
exporting, by the computing device, the optimal design parameters.

2. The method of claim 1, where determining the optimal design parameters includes determining a size of a placement fixture.

3. The method of claim 1, where determining the optimal design parameters includes determining a number of placement fixtures.

4. The method of claim 1, where determining the optimal design parameters includes determining a shape of a placement fixture.

5. The method of claim 1, where determining the optimal design parameters includes determining a location of a placement fixture.

6. The method of claim 1, where determining the optimal design parameters includes iterating over a plurality of guesses for the optimal design parameters.

7. The method of claim 1, further comprising generating a plurality of designs for the prosthetic, each of the plurality of designs including a different permutation of design parameters.

8. The method of claim 1, wherein
receiving the patient specific data comprises receiving a scan of a patient's anatomy; and
the method further comprises extracting design constraints for the prosthetic from the scan.

9. The method of claim 1, wherein exporting the optimal design parameters includes transmitting the optimal design parameters to an automated manufacturing device.

10. The method of claim 1, wherein determining the optimal design parameters includes generating the machine learning model by:
receiving, by a computing device, a plurality of input parameters, each of the plurality of input parameters corresponding to a parameter of at least one of a plurality of prosthetic designs;
generating, by the computing device, an initial guess for the machine learning model based on the plurality of input parameters;
optimizing, by the computing device, parameters of the machine learning model using the initial guess for the machine learning model and the plurality of input parameters; and
exporting, by the computing device, the machine learning model having optimized parameters.

11. The method of claim 10, wherein optimizing the parameters of the machine learning model comprises training the machine learning model using a set of finite element analyses performed on models of the prosthetic and at least a subset of the input parameters.

12. The method of claim 10, wherein the plurality of input parameters comprises patient data for a plurality of patients.

13. The method of claim 10, further comprising dividing the plurality of input data into a training subset used in optimizing the parameters of the machine learning model and a validation subset used for confirming a validity of the machine learning model.

14. The method of claim 10, wherein receiving the plurality of input parameters comprises:
receiving a scan of a patient's anatomy for a plurality of patients; and
extracting at least one of the input parameters from the scan of the patient's anatomy for each of the plurality of patients.
